(19) **Europäisches Patentamt European Patent Office Office européen des brevets**

(11) **EP 4 185 263 B1**

(12) **EUROPEAN PATENT SPECIFICATION**

(45) Date of publication and mention of the grant of the patent:
**10.06.2026 Bulletin 2026/24**

(21) Application number: **20758321.2**

(22) Date of filing: **23.07.2020**

(51) International Patent Classification (IPC):
*A61K 8/19* (2006.01)  *A61K 8/29* (2006.01)
*A61K 8/73* (2006.01)  *A61K 8/891* (2006.01)
*A61K 8/02* (2006.01)  *A61Q 1/02* (2006.01)
*A61Q 1/04* (2006.01)  *A61Q 1/10* (2006.01)

(52) Cooperative Patent Classification (CPC):
**A61K 8/29; A61K 8/0241; A61K 8/19; A61K 8/732; A61K 8/891; A61Q 1/02; A61Q 1/04; A61Q 1/10;**
A61K 2800/43; A61K 2800/612; A61K 2800/614

(86) International application number:
**PCT/IB2020/000644**

(87) International publication number:
**WO 2022/018474 (27.01.2022 Gazette 2022/04)**

(54) **SURFACE-TREATED PIGMENT AND A COSMETIC COMPOSITION CONTAINING THE SAME**

OBERFLÄCHENBEHANDELTES PIGMENT UND KOSMETISCHE ZUSAMMENSETZUNG DAMIT

PIGMENT TRAITÉ EN SURFACE ET COMPOSITION COSMÉTIQUE LE CONTENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**31.05.2023 Bulletin 2023/22**

(73) Proprietor: **Chanel Parfums Beauté**
**92200 Neuilly-sur-Seine (FR)**

(72) Inventors:
- **YAMAKI, Hideyuki**
  **Funabashi-shi Chiba, 273-0045 (JP)**
- **KUROMIYA, Tomomi**
  **Funabashi-shi Chiba, 273-0045 (JP)**

- **NAGAHIRO, Chikako**
  **Funabashi-shi Chiba, 273-0045 (JP)**
- **ITO, Hisao**
  **Funabashi-shi Chiba, 273-0045 (JP)**

(74) Representative: **Plasseraud IP**
**104 Rue de Richelieu**
**CS92104**
**75080 Paris Cedex 02 (FR)**

(56) References cited:
**WO-A1-2018/029209**

- **DATABASE WPI Week 201173, Derwent World Patents Index; AN 2011-N36257, XP002802697**
- **DATABASE WPI Week 201914, Derwent World Patents Index; AN 2019-15710M, XP002802698**

Description

## FIELD OF THE INVENTION

The present invention relates to a surface-treated pigment obtained by coating the pigment surface with at least a dextrin fatty ester and at least a silicone acrylate copolymer, and a cosmetic composition containing the same having improved stability properties, as regards to color and brightness overtime.

## BACKGROUND OF THE INVENTION

Makeup foundations are used in the cosmetic field to provide a uniform and stable complexion, especially in terms of color and brightness, over a long period of time.

Most of these cosmetic formulations include pigments for providing desired visual properties such as opacity and color, and/or special effects such as pearlescence and iridescence.

However, makeup foundations, especially fluid foundations often have the drawback of color changes over time, such as changing to darker or reddish shade upon application and drying on the skin mostly due to evaporation of the liquid phase (water and volatile oils). These color changes and the migration of the makeup are disappointing for the consumer who wishes to obtain a uniform and stable shade on the skin over a long period of time.

Accordingly, it is important that the color of the compositions remain stable once applied to the skin, i.e., do not fade or discolor or migrate or change.

One principal cause for the evolution of the color of the compositions once applied on the skin is the difference of color between pigments when dispersed in a wet formulation compared to its dry form.

In the past, efforts to improve the color stability of pigments have involved surface treating of pigments and powders. Accordingly, over the years, many compositions and methods have been developed to surface treat cosmetic powders, and in particular pigments, in order to overcome their stability issues. Conventionally, different hydrophobized pigment coatings have been developed for a higher dispersibility in the oily phase and to avoid wetting said pigments by the oils like OTS (octadecyltrichlorosilane) coating, NAI (disodium stearoyl glutamate) coating, ASI (combination of isopropyl Titanium Triisostearate (ITT) and Sodium Lauroyl Aspartate) coating, ASL ( sodium lauroyl glutamate lysine, magnesium chloride) coating, PF (perfluoro alkyle phosphates) coating, FHS (perfluorooctyltriethoxysilane) coating.

More recently, surface treatment with dextrin fatty esters has been described, such as in document JP,5695331 in order to provide surface-treated pigment that has good dispersibility and adhesion to the skin, and has excellent makeup sustainability. However, these surface-treated pigment did not provide sufficiently good results in terms of shade and color stability of the applied makeup over time.

The application JP2011/213662 discloses a surface-treated powdery material prepared by surface-coating its surface with a dextrin fatty acid ester. The application KR2018/0136315 discloses an acrylate/dimethicone copolymer coated boron nitride. The international application WO2018/029209 discloses the use of dextrin isostearate and acrylate/dimethicone copolymer as coating agent.

## TECHNICAL PROBLEM

The present invention is made in view of the above problems and an object of the present invention is to provide cosmetic pigments having improved stability properties, as regards to color and brightness overtime, their manufacturing method and cosmetic compositions containing such surface-treated pigments, said compositions thus showing improved stability of their color and shade over time.

## SOLUTION TO PROBLEM

The inventors conducted an intensive research to solve the aforementioned problem. As a result, they have found that the one solution could be to ensure a wetting of the pigment in a specific oil through a first coating, and an entrapment of said wetted pigments through another specific second coating. Thus, the present invention relates to a treated pigment obtained by surface treating a pigment with a dextrin fatty ester and a silicone acrylate copolymer.

Without wishing to be bound by any theory, it seems that the pigments according to the present invention are wetted by the dextrin fatty ester; the oil is hence adsorbed around the pigments. As the pigments' surface is also treated with a silicone acrylate copolymer, said silicone acrylate copolymer prevents elution of the dextrin fatty ester. The oil is thus maintained around the pigment to keep the pigment in a wet state for a long period of time.

Hence, the present invention allows providing surface-treated pigments and cosmetic compositions containing the same, having improved stability properties, as regards to color and brightness overtime. The cosmetic compositions according to the invention remain stable as regard to the color and do not fade or discolor or migrate or change.

## SUMMARY OF THE INVENTION

The present invention is defined by the appended claims.

Thus, **in a first aspect,** the invention relates to a surface-treated pigment whose surface-treatment comprises at least a dextrin fatty acid ester and at least a silicone acrylate copolymer,

wherein the pigment is a mineral pigment, and
wherein the surface-treatment comprises 1 to 15 % by weight, relative to the total weight of the composition, of the dextrin fatty acid ester and 1 to 15% by weight relative to the total weight of the composition, of the silicone acrylate copolymer.

In **a second aspect,** the invention relates to a process of preparing a surface-treated pigment according to the invention comprising:

- dissolving a dextrin fatty ester and a silicone acrylate copolymer in a solvent to form a slurry;
- adding the slurry to pigments under stirring;
- removing the solvent;
- pulverizing and heating the pigments in order to obtain surface-treated pigments.

Advantageously, the surface-treated pigment have improved color and brightness properties so that the surface-treated pigments as well as cosmetic compositions which include them remain stable as regard to the color and do not fade or discolor or migrate or change.

Hence, in a **third aspect,** the invention relates to cosmetic composition comprising a surface-treated pigment according to the present invention, and a cosmetically acceptable medium.

In a **fourth aspect,** the invention relates to the use of surface-treated pigment according to the present invention for improving the stability of the color and brightness of the make-up.

## DETAILED DESCRIPTION OF THE INVENTION

### *Surface-treated pigments*

In a **first aspect,** the present invention relates to a surface-treated pigment characterized in that its surface-treatment comprises at least a dextrin fatty acid ester and at least a silicone acrylate copolymer,

wherein the pigment is a mineral pigment, and
wherein the surface-treatment comprises 1 to 15 % by weight, relative to the total weight of the composition, of the dextrin fatty acid ester and 1 to 15% by weight relative to the total weight of the composition, of the silicone acrylate copolymer.

### *Dextrin fatty acid ester*

Dextrin fatty acid ester is described for example in the European patent application EP2537865. Dextrin fatty acid ester is prepared by esterification between dextrin and fatty acids. The degree of substitution of the dextrin by the fatty acids is 1.0 to 3.0, preferably 1.2 to 2.8, per glucose unit.

The dextrin used in the dextrin fatty acid ester is preferably dextrin having an average degree of glucose polymerization of 3 to 150, particularly, 10 to 100. The dextrin may have any of linear, branched, and cyclic sugar chains.

The fatty acids used in the dextrin fatty acid ester inevitably comprise one or more saturated branched fatty acids having 4 to 26 carbon atoms and may further comprise one or more fatty acids selected from the group consisting of saturated linear fatty acids having 2 to 22 carbon atoms, unsaturated linear or branched fatty acids having 6 to 30 carbon atoms, and saturated or unsaturated cyclic fatty acids having 6 to 30 carbon atoms (hereinafter, these fatty acids other than the saturated branched fatty acids having 4 to 26 carbon atoms are also collectively referred to as "the other fatty acids").

The compositional ratios of the fatty acids are more than 50 mol% and 100 mol% or less, preferably 55 mol% or more and 100 mol% or less, based on the total amount of the fatty acids, of one or more saturated branched fatty acids having 4 to 26 carbon atoms and 0 mol% or more and less than 50 mol%, preferably 0 mol% or more and 45 mol% or less, based on the total amount of the fatty acids, of the other fatty acids.

Examples of the saturated branched fatty acids having 4 to 26 carbon atoms, used include isobutyric acid, isovaleric acid, 2-ethylbutyric acid, ethylmethylacetic acid, isoheptanoic acid, 2-ethylhexanoic acid, isononanoic acid, isodecanoic acid, isotridecanoic acid, isomyristic acid, isopalmitic acid, isostearic acid, isoarachic acid, and isohexacosanoic acid. One or more of these fatty acids can be selected or combined appropriately for use.

Preferably, the fatty acid is isostearic acid. As used herein, isostearic acid means one branched stearic acid or a mixture of two or more branched stearic acids.

Examples of the saturated linear fatty acids having 2 to 22 carbon atoms, used in the present invention, include acetic acid, caprylic acid, capric acid, lauric acid, myristic acid, palmitic acid, stearic acid, arachic acid, and behenic acid. One or more of these fatty acids can be selected or combined appropriately for use.

Examples of the unsaturated linear or branched fatty acids having 6 to 30 carbon atoms, used in the present invention, include: unsaturated monoenoic fatty acids such as cis-4-decenoic (obtusilic) acid, 9-decenoic (caproleic) acid, cis-4-dodecenoic (linderic) acid, cis-4-tetradecenoic (tsuzuic) acid, cis-5-tetradecenoic (physeteric) acid, cis-9-tetradecenoic (myristoleic) acid, cis-6-hexadecenoic acid, cis-9-hexadecenoic (palmitoleic) acid, cis-9-octadecenoic (oleic) acid, trans-9-octadecenoic acid (elaidic acid), cis-11-octadecenoic (asclepinic) acid, cis-11-eicosenoic (gondoic) acid, cis-17-hexacosenoic (ximenic) acid, and cis-21-triacontenoic (lumequeic) acid; and unsaturated polyenoic fatty acids such as sorbic acid, linoleic acid, hiragonic acid, punica acid, linolenic acid, $\gamma$-linolenic acid, moroctic acid, stearidonic acid, arachidonic acid, EPA, clupanodonic acid, DHA, nisinic acid, stearolic acid, crepenynic acid, and ximenynic acid.

The saturated or unsaturated cyclic fatty acids having 6 to 30 carbon atoms, used in the present invention, mean saturated or unsaturated fatty acids having a cyclic structure in at least a portion of the basic skeleton and having 6 to 30 carbon atoms. Examples thereof include 9,10-methylene-9-octadecenoic acid, aleprylic acid, alepric acid, gorlic acid, $\alpha$-cyclopentyl acid, $\alpha$-cyclohexyl acid, $\alpha$-cyclopentylethyl acid, $\alpha$-cyclohexylmethyl acid, $\omega$-cyclohexyl acid, 5(6)-carboxy-4-hexyl-2-cyclohexene-1-octanoic acid, malvalic acid, sterculynic acid, hydnocarpic acid, and chaulmoogric acid.

In a preferred embodiment, the dextrin fatty acid ester is a dextrin isostearate (for example, a product commercially available from CHIBA FLOUR MILLING CO., Ltd, under the commercial name such as UNIFILMA HVY).

*Silicone acrylate copolymer*

Silicone acrylate copolymer can be chosen among Acrylates/polytrimethylsiloxymethacrylate copolymer, Acrylates/ethylhexylacrylate/dimethicone methacrylate copolymer, Styrene/acrylates/dimethicone acrylate crosspolymer and acrylates/dimethicone copolymer.

In a preferred embodiment, the silicone acrylate is an acrylates/dimethicone copolymer. For example, acrylates/dimethicone copolymers used are those produced by DAITO KASEI KOGYO CO., LTD under the product name "ASC treatment" and the following commercial references: ASC-2 TiO2 CR-50; ASC-5 RED R-516L; ASC-7 YELLOW LL-100P; ASC-2 BLACK BL-100.

*Surface-treated pigment*

As used herein, the term **"pigment"** means any pigments which can be used regardless of their forms (spherical, bar-like, needle-like, plate-like, infinite, scale-like, spindle-shaped etc.), particle sizes (haze particles, fine particles, as small as pigment class etc.), or particle structures (porous, imperforate etc.).

As used herein, the term **"pigment"** includes white or coloured, mineral particles, insoluble in aqueous medium, intended to colour and/or opacify the composition.

As disclosed herein, the pigment may be an organic pigment. The organic pigment may in particular be chosen from the compounds nitroso, nitro, azo, xanthene, quinoline, anthraquinone, phthalocyanine, metal complex type, isoindolinone, isoindoline, quinacridone, perinone, perylene, diketopyrrolopyrrole, thioindigo, dioxazine, triphenylmethane, quinophthalone.

The organic pigment as disclosed herein may be chosen for example from carmine, carbon black, aniline black, melanin, azo yellow, quinacridone, phthalocyanine blue, sorghum red, blue pigments coded in the Color Index under references C1 42090, 69800, 69825, 73000, 74100, 74160, the yellow pigments coded in the Color Index under references CI 11680, 11710, 15985, 19140, 20040, 21100, 21108, 47000, 47005, the green pigments coded in the Color Index under references CI 61565, 61570, 74260, the orange pigments coded in the Color Index under references CI 11725, 15510, 45370, 71105, the red pigments coded in the Color Index under references CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, or pigments obtained by oxidative polymerisation of indole or phenolic derivatives, as described in patent FR 2679771.

These pigments as disclosed herein may also be in the form of a composite pigment as described in patent EP1184426. A composite pigment may be composed of particles comprising an inorganic core at least partially coated with an organic pigment and at least one binder which fixes the organic pigments to the core.

The pigment as disclosed herein can also be a lacquer. Lacquer means insolubilized dyes adsorbed on insoluble particles, the whole thus obtained remaining insoluble during use. Examples of lacquers include the product known as: D & C Red 7 (CI 15 850:1).

The pigment according to the present invention is a mineral pigment. A mineral pigment is any pigment that meets the definition of the Ullmann Encyclopedia in the chapter Inorganic Pigment. Mineral pigments are chosen among zirconium or cerium oxide, zinc oxide, iron oxide (black, yellow or red) or chromium oxide, manganese violet, ultramarine blue, chromium hydrate and ferric blue, titanium dioxide, metal powders such as aluminum powder and copper powder. The following mineral pigments can also be used: $Ti_2O_5$, $Ti_3O_5$, $Ti_2O_3$, $TiO$, $ZrO_2$ in mixture with $TiCO_2$, $ZrO_2$, $Nb_2O_5$, $CeO_2$, $ZnS$.

In a preferred embodiment, the mineral pigment is an iron oxide (black, yellow or red).

For example, iron oxide used can be those produced by Sensient Technologies Corporation under the product name UNIPURE YELLOW LC 182 HLC QUAL. STERILE, UNIPURE RED LC 381 HLC QUALITE STERILE.

The size of the pigment useful in the context of the present invention is generally between 100 nm and 10 $\mu$m, preferably between 200 nm and 5 $\mu$m, and more preferably between 300 nm and 1 $\mu$m.

The surface treatment comprises 1 to 15 % by weight, relative to the total weight of the composition, of the dextrin fatty ester and 1 to 15 % by weight, relative to the total weight of the composition, of the silicone acrylate copolymer.

In an embodiment, the surface treatment comprises 1 to 12 % by weight relative to the total weight of the composition, of the dextrin fatty ester preferably from 2 to 10% by weight, relative to the total weight of the composition, and 1 to 10 % by weight, relative to the total weight of the composition, of the silicone acrylate copolymer, preferably from 2 to 7% by weight of the total weight of the composition.

### Wet state

One principal cause for the evolution of the color of the compositions once applied on the skin is the difference in color between pigments when dispersed in a wet formulation compared to its dry form. According to the invention, one solution is to ensure a wetting of the pigment in specific oil through a first coating, and an entrapment of said wetted pigments through another specific second coating. The oil is thus maintained around the pigment to keep the pigment in a wet state for a long period of time.

As used herein "**wet state**" means maintaining oil around the pigment

This particular coating allows providing surface-treated pigments having improved stability properties as regards to color and brightness over time.

### Process

In a **second aspect,** the present invention also relates to a process of preparing a surface-treated pigment according to the invention comprising:

- dissolving a dextrin fatty ester and a silicone acrylate copolymer in a solvent to form a slurry;
- adding the slurry to pigments under stirring;
- removing the solvent;
- pulverizing and heating the pigments in order to obtain surface-treated pigments.

Typically, the solvent used is chosen among acetone, isobutyl alcohol, isopropyl alcohol, isopentyl alcohol, methanol, ethanol and hexane. Preferably, the solvent is hexane.

Typically, 5 to 15 parts by weight, preferably 6 to 12, preferably 8 to 10 parts by weight of dextrin fatty ester and 5 to 15 parts by weight, preferably 6 to 12, preferably 8 to 10 parts by weight of silicone acrylate copolymer are dissolved in 10 to 20 parts by weight, preferably 12 to 18, preferably 14 to 16 parts by weight of solvent, and then added to 80 to 95 parts by weight, preferably 82 to 90 parts by weight of pigments.

After forming a coating layer with dextrin fatty ester and silicone acrylate copolymer, the solvent is removed and the pigments are pulverized and heated in order to obtain surface-treated pigments according to the present invention.

### Cosmetic composition

In the **third aspect,** the present invention relates to a cosmetic composition comprising a surface-treated pigment according to the present invention, and a cosmetically acceptable medium.

The surface-treated pigment is the surface-treated pigment as previously described. All the previously described embodiments are encompassed in this aspect.

As used herein the term "cosmetically acceptable medium" refers to a medium that is suitable for use in contact with the skin without undue adverse side effects (such as toxicity, irritation, allergic response, and the like).

*Cosmetically acceptable component*

As long as the purpose and effect of the present invention are not impaired, cosmetic composition of the present invention can further contain a cosmetically acceptable component in addition to the surface-treated pigment of the present invention.

Examples of the cosmetically acceptable components include powder components other than the surface-treated pigment of the present invention, a liquid oil, a solid fat, a wax, a hydrocarbon, a higher fatty acid, a higher alcohol (preferably an alcohol having 6 or more carbon atoms, and more preferably an alcohol having 10 or more carbon atoms), a synthetic ester oil, a silicone oil, a surfactant, a co-surfactant, a moisturizing agent, a film forming agent, a thickener, a gelling agent, a metal sequestering agent, a lower alcohol, a polyhydric alcohol, a monosaccharide, an oligosaccharide, an amino acid, a plant extract, an organic amine, a polymer emulsion, an antioxidant, an antioxidant aid, a vitamin, an antibacterial agent, an anti-inflammatory agent, a water-soluble polymer, a skin whitening agent, a UV absorber such as organic and/or inorganic sunscreen, an antiseptic agent, a skin softener, an antiaging agent, an anti-pollution agent, a keratolytic agent, a pH adjustor, a buffer, a perfume and water.

Any of the above-escribed components may be suitably selected and blended depending on a desired formulation and a product form. The blending amount of the components is no particularly limited as long as it can be used without departing from the purpose of the present invention. The blending amount is suitably selected depending on a formulation, a product form, etc.

Examples of the powder components include inorganic powders, such as talc, kaolin, mica, sericite, white mica, gold mica, a synthetic mica, red mica, black mica, vermiculite, magnesium carbonate, calcium carbonate, aluminum silicate, barium silicate, calcium silicate, magnesium silicate, strontium silicate, a metal tungstate, silica, zeolite, barium sulfate, magnesium sulfate, calcined calcium sulfate (calcined gypsum), calcium phosphate, fluorine apatite, hydroxyapatite, ceramic powder, metallic soap (e.g. zinc myristate, calcium palmitate, aluminum stearate, magnesium stearate and boron nitride); organic powders, such as polyamide resin powder (nylon powder), polyethylene powder, polymethyl methacrylate powder, polystyrene powder, styrene/acrylic acid copolymer resin powder, benzoguanamine resin powder, polytetrafluoroethylene powder and cellulose powder; metal powder pigments, such as aluminum powder and copper powder; organic pigments, such as a zirconium-, barium-, or aluminum-lakes; and natural colors, such as chlorophyll and β-carotene. Note that the powder components may be hydrophobized.

Examples of the liquid oil include avocado oil, camellia oil, turtle oil, macadamia nut oil, corn oil, mink oil, olive oil, rapeseed oil, yolk oil, sesame oil, persic oil, wheat germ oil, camellia kissi oil, castor oil, linseed oil, safflower oil, cotton seed oil, perilla oil, soybean oil, peanut oil, tea seed oil, Torreya seed oil, rice bran oil, Chinese tung oil, Japanese tung oil, jojoba oil, germ oil, and triglycerin.

Examples of the solid fat include cacao butter, coconut oil, horse tallow, hardened coconut oil, Japan tallow kernel oil, hardened oil, Japan tallow, and hardened castor oil.

Examples of the wax include bees wax, candelilla wax, cotton wax, carnauba wax, bayberry wax, Chinese insect wax, montan wax, bran wax, lanolin, kapok wax, lanolin acetate, liquid lanolin, sugar cane wax, lanolin fatty acid isopropyl ester, hexyl laurate, reduction lanolin, jojoba wax, hard lanolin, shellac wax, POE lanolin alcohol ether, POE lanolin alcohol acetate, POE cholesterol ether, lanolin fatty acid polyethylene glycol, and POE hydrogenated lanolin alcohol ether.

Examples of the hydrocarbon oil include liquid paraffin, ozokerite, squalane, pristane, paraffin, ceresin, squalene, vaseline, microcrystalline wax, and hydrogenated polydecene.

Examples of the higher fatty acid include lauric acid, myristic acid, palmitic acid, stearic acid, behenic acid, oleic acid, undecylenic acid, tall oil acid, isostearic acid, linoleic acid, linolenic acid, eicosapentaenoic acid (EPA), and docosahexaenoic acid (DHA).

Examples of the higher alcohol include linear alcohols, such as lauryl alcohol, cetyl alcohol, stearyl alcohol, behenyl alcohol, myristyl alcohol, oleyl alcohol, and cetostearyl alcohol; branched alcohols, such as monostearyl glycerin ether (batyl alcohol), 2-decyltetradecanol, lanolin alcohol, cholesterol, phytosterol, hexyldodecanol, isostearyl alcohol, and octyldodecanol.

Examples of the synthetic ester oil include tripropylene glycol dineopentanoate, isononyl isononanoate, isotridecyl isononanoate, isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid ester, N-alkylglycol monoisostearate, neopentyl glycol dicaprylate, diisostearyl malate, glycerin di-2-heptylundecanoate, trimethylolpropane tri-2-ethylhexanoate, trimethylolpropane triisostearate, pentaerythritol tetra-2-ethylhexanoate, glycerin tri-2-ethylhexanoate, glycerin trioctanoate, glycerin triisopalmitate, trimethylolpropane triisostearate, cetyl 2-ethylhexanoate, 2-ethylhexyl palmitate, glycerin trimyristate, glyceride tri-2-heptylundecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, triethyl citrate, bis-behenyl/isostearyl/phytosteryl dimer dilinoleyl dimer dilinoleate, phytosteryl/behenyl/octyldodecyl/isostearyl lauroyl glutamate, and tri(caprylic acid/capric acid) glyceryl.

Examples of the silicone oil include a chain polysiloxane, such as dimethicone, methyl trimethicone, methylphenylpolysiloxane and diphenylpolysiloxane; a cyclic polysiloxane, such as octamethyl cyclotetrasiloxane, decamethyl cyclopentasiloxane and dodecamethyl cyclohexasiloxane; a silicone resin forming a 3D net structure; a silicone rubber; a silicone elastomer; various modified polysiloxanes, such as amino-modified polysiloxane, polyether-modified polysiloxane, alkyl-modified polysiloxane and fluorine-modified polysiloxane.

Examples of the silicone resin include acrylates/polytrimethylsiloxymethacrylate copolymers commercially available from DOW CORNING Corporation, under the trade name of, for example, DC FA 4001 and DC FA 4002, and the like. Other examples of the silicone resin include trimethylsiloxysilicate/dimethiconol crosspolymers commercially available from DOW CORNING Corporation, under the trade name of, for example, DC 7-4411.

Examples of silicone elastomers include non-emulsifying organopolysiloxane elastomers or emulsifying organosiloxane elastomers. Examples of the non-emulsifying organopolysiloxane elastomers include dimethicone/vinyl dimethicone crosspolymers, lauryl dimethicone/vinyl dimethicone crosspolymers, and the like.

The dimethicone/vinyl dimethicone crosspolymers include products commercially available from DOW CORNING Corporation, Midland, Michigan, under the trade name of, for example, DC 9040 and DC 9045; products commercially available from MOMENTIVE Performance Materials Inc. under the trade name of SFE 839 and the Velvasil series products; products commercially available from SHIN-ETSU Chemical Co., Ltd. under the trade name of, for example, KSG-15, KSG-16, and KSG-18 ([dimethicone/phenyl vinyl dimethicone crosspolymer]); and Gransil (TM) series products from GRANT INDUSTRIES, Inc.

The lauryl dimethicone/vinyl dimethicone crosspolymers include products commercially available from SHIN-ETSU Chemical Co., Ltd. under the trade name of, for example, KSG-31, KSG-32, KSG-41, KSG-42. KSG-43, and KSG-44.

Examples of the emulsifying organosiloxane elastomers include polyalkoxylated silicone elastomers, polyglycerolated silicone elastomers, or the like.

The polyalkoxylated silicone elastomers include products commercially available from DOW CORNING Corporation under the trade name of, for example, DC9010 and DC9011; products commercially available from SHIN-ETSU Chemical Co., Ltd. under the trade name of, for example, KSG-20, KSG-21, KSG-30, KSG-31, KSG-32, KSG-33, KSG-210, KSG-310, KSG-320, KSG-330, KSG-340, and X-226146.

The polyglycerolated silicone elastomers include products commercially available from SHIN-ETSU Chemical Co., Ltd. under the trade name of, for example, KSG-710, KSG-810, KSG-820, KSG-830, KSG-840, KSG-31, KSG-32, KSG-41, KSG-42, KSG-43, and KSG-44. In addition, examples of silicone elastomers into which 2 types of branches, i.e., a silicone chain and an alkyl chain have been introduced include products commercially available from SHIN-ETSU Chemical Co., Ltd. under the trade name of, for example, KSG-042Z, KSG-045Z, KSG-320Z, KSG-350Z, KSG-820Z, and KSG-850Z.

Silicone elastomers comprising a polyalkyl ether group as pendant or cross linked may also included as components in the cosmetic composition of the present invention. Particularly suitable silicone elastomers comprising a polyalkyl ether group include compounds with the International Nomenclature of Cosmetic Ingredients (INCI) name: bis-vinyldimethicone/bis-isobutyl PPG-20 crosspolymer, bis-vinyldimethicone/PPG-20 crosspolymer, dimethicone/bis-isobutyl PPG-20 crosspolymer, dimethicone/PPG-20 crosspolymer, and dimethicone/bis-sec butyl PPG-20 crosspolymer. Such cross-linked elastomers are available from Dow Corning Corporation under the experimental names of SOEB-1, SOEB-2, SOEB-3 and SOEB-4, etc., and under the proposed commercial name of DC EL-8052 IH Si Organic Elastomer Blend, etc. The elastomer particles are supplied pre-swollen in the respective solvents, isododecane (for SOEB-1 and SOEB-2), isohexadecane (for SOEB-3), and isodecyl neopentanoate (for SOEB-4).

Examples of the surfactant include a lipophilic nonionic surfactant and a hydrophilic nonionic surfactant.

Examples of the lipophilic nonionic surfactant include a sorbitan fatty acid ester, such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate and diglycerol sorbitan tetra-2-ethylhexylate; a glycerin polyglycerin fatty acid, such as glycerin mono-cotton seed oil fatty acid, glycerin monoerucate, glycerin sesquioleate, glycerin monostearate, glycerin $\alpha,\alpha'$-oleate pyroglutamate, and glycerin monostearate malate; a propylene glycol fatty acid ester such as monostearate propylene glycol; a hardened castor oil derivative; and a glycerin alkyl ether.

Examples of the hydrophilic nonionic surfactant include a POE-sorbitan fatty acid ester, such as POE-sorbitan monooleate, POE-sorbitan monostearate, POE-sorbitan monooleate and POE-sorbitan tetraoleate; a POE sorbitol fatty acid ester, such as POE-sorbitol monolaurate, POE-sorbitol monooleate, POE-sorbitol pentaoleate and POE-sorbitol monostearate; a POE-glycerin fatty acid ester, such as POE-glycerin monostearate, POE-glycerin monoisostearate and POE-glycerin triisostearate; a POE-fatty acid ester, such as POE-monooleate, POE-distearate, POE-monodioleate and ethylene glycol distearate; a POE-alkyl ether, such as POE-lauryl ether, POE-oleyl ether, POE-stearyl ether, POE-behenyl ether, POE-2-octyldodecyl ether and POE-cholestanol ether; a Pluronic type surfactant (e.g., Pluronic); a POE-POP-alkyl

**EP 4 185 263 B1**

ether, such as POE-POP-cetyl ether, POE-POP-2-decyltetradecyl ether, POE-POP-monobutyl ether, POE-POP-hydrogenated lanolin and POE-POP-glycerin ether.

Examples of the co-surfactants include higher alcohols. Among them, linear alcohols such as lauryl alcohol, cetyl alcohol, stearyl alcohol, behenyl alcohol, myristyl alcohol, oleyl alcohol, cetostearyl alcohol, and the like, are preferable. Cetyl alcohol is particularly preferable.

Examples of the metal sequestering agent include 1-hydroxyethane-1,1-diphosphonic acid; 1-hydroxyethane-1,1-diphosphonic acid tetrasodium salt; disodium edetate; trisodium edetate; tetrasodium edetate; sodium citrate; sodium polyphosphate; sodium metaphosphate; gluconic acid; phosphoric acid; citric acid; ascorbic acid; succinic acid; edetic acid; and trisodium ethylenediamine hydroxyethyl triacetate.

Examples of the lower alcohol include ethanol, propanol, isopropanol, isobutyl alcohol, and t-butyl alcohol.

Examples of the polyhydric alcohol include a dihydric alcohol, such as ethylene glycol, propylene glycol, pentylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol and octylene glycol; a trihydric alcohol, such as glycerin and trimethylolpropane; a tetrahydric alcohol such as pentaerythritol (e.g., 1,2,6-hexanetriol); a pentahydric alcohol such as xylitol; a hexahydric alcohol, such as sorbitol and mannitol; a polyhydric alcohol polymer, such as diethylene glycol, dipropylene glycol, triethylene glycol, polypropylene glycol and tetraethylene glycol; a dihydric alcohol alkyl ether, such as ethylene glycol monomethyl ether and ethylene glycol monoethyl ether; a dihydric alcohol alkyl ether, such as diethylene glycol monomethyl ether, diethylene glycol monoethyl ether and diethylene glycol monobutyl ether; a dihydric alcohol ether ester, such as ethylene glycol monomethyl ether acetate and ethylene glycol monoethyl ether acetate; a glycerin monoalkyl ether, such as chimyl alcohol, selachyl alcohol and batyl alcohol; and a sugar alcohol, such as sorbitol, maltitol, maltotriose, mannitol, sucrose, erythritol, glucose, fructose, starch sugar, maltose, xylitose, and a reduced alcohol of a starch sugar.

Examples of the monosaccharide include a triose, such as D-glyceryl aldehyde and dihydroxyacetone; a tetrose, such as D-erythrose, D-erythrulose, D-threose and erythritol; a pentose, such as L-arabinose, D-xylose, L-lyxose, D-arabinose, D-ribose, D-ribulose, D-xylulose and L-xylulose; a hexose, such as D-glucose, D-talose, D-psicose, D-galactose, D-fructose, L-galactose, L-mannose and D-tagatose; a heptose, such as aldoheptose and heprose; an octose such as octurose; a deoxy sugar, such as 2-deoxy-D-ribose, 6-deoxy-L-galactose and 6-deoxy-L-mannose; an amino sugar, such as D-glucosamine, D-galactosamine, sialic acid, amino uronic acid and muramic acid; a uronic acid, such as D-glucuronic acid, D-mannuronic acid, L-guluronic acid, D-galacturonic acid and L-iduronic acid.

Examples of the oligosaccharide include sucrose, lactose, maltose, trehalose, cellobiose, gentiobiose, umbilicin, raffinose, gentianose, maltotriose, melezitose, planteose, unbelliferose, stachyose, and verbascose.

Examples of the amino acid include a neutral amino acid, such as threonine and cysteine; and a basic amino acid such as hydroxylysine. Further, as an amino acid derivative, for example, sodium acyl sarcosinate (sodium lauroyl sarcosinate), acyl glutamate, sodium acyl β-alanine, glutathione, and pyrrolidone carboxylic acid may be exemplified.

Examples of the organic amine include monoethanolamine, diethanolamine, triethanolamine, morpholine, triisopropanolamine, 2-amino-2-methyl-1,3-propanediol, and 2-amino-2-methyl-1-propanol.

Examples of the polymer emulsion include an acrylic resin emulsion, a poly(ethyl acrylate) emulsion, an acrylic resin solution, a poly(alkyl acrylate) emulsion, a poly(vinyl acetate) resin emulsion, and a natural rubber latex.

Examples of the vitamin include vitamins A, B1, B2, B6, C and E and derivatives thereof, pantothenic acid and derivatives thereof and biotin.

Examples of the antioxidants include ascorbic acid and its derivatives such as ascorbyl palmitate, ascorbyl tetraisopalmitate, ascorbyl glucoside, magnesium ascorbyl phosphate, sodium ascorbyl phosphate and ascorbyl sorbate; tocopherol and its derivatives, such as tocopherol acetate, tocopherol sorbate, and other esters of tocopherol; dibutyl hydroxytoluene (BHT) and butyl hydroxyanisole (BHA); gallic acid ester; phosphoric acid; citric acid; maleic acid; malonic acid; succinic acid; fumaric acid; cephalin; a hexametaphosphate; phytic acid; ethylenediaminetetraacetic acid; and plant extracts, for instance from Chondrus crispus, Rhodiola, Thermus thermophilus, mate leaves, oak wood, kayu rapet bark, sakura leaves and ylang ylang leaves.

Examples of the moisturizing agent include polyethylene glycol; propylene glycol; dipropylene glycol; glycerin; 1,3-butylene glycol; xylitol; sorbitol; maltitol; mucopolysaccharides such as chondroitin sulfuric acid; hyaluronic acid; mucoitinsulfuric acid; caronic acid; atelo-collagen; cholesteryl-12-hydroxystearate; bile salt; a main component of NMF (natural moisturizing factor), such as a pyrrolidone carboxylic acid salt and a lactic acid salt; amino acids such as urea, cysteine and serine; short-chain soluble collagen; a diglycerin (EO) PO addition product; homo- or copolymers of 2-methacryloyloxyethylphosphorylcholine commercially available from NOF CORPORATION under the trade name of, for example, Lipidure HM and Lipidure PBM; panthenol; allantoin; PEG/PPG/Polybutylene Glycol-8/5/3 Glycerin commercially available from NOF CORPORATION under the trade name of Wilbride S 753; Trimethylglycine commercially available from Asahi KASEI Chemicals Corporation under the trade name of AMINOCOAT; and various plant extracts such as Castanea sativa extracts, hydrolyzed hazelnut proteins, Polianthes tuberosa polysaccharides, Argania spinosa kernel oil, and an extract of pearl containing conchiolin commercially available from Maruzen Pharmaceuticals Co. LTD. under the trade name of Pearl Extract [®].

Examples of the skin softener include glyceryl polymethacrylate, methyl gluceth-20 and the like.

Examples of the antiaging agent include acyl amino acids (specifically, products commercially available from SEDERMA under the trade name of Maxilip, Matrixyl 3000 or Biopeptide CL, or product commercially available from SEPPIC under the trade name of Sepilift); Pisum sativum extracts; hydrolyzed soy proteins; methylsilanol mannuronate; hydrolyzed cucurbita pepo seedcake; Scenedesmus extract; and the like.

Examples of the anti-pollution agents include Moringa pterygosperma seed extracts (specifically, product commercially available from LSN under the trade name of Purisoft); Shea butter extract (specifically, products commercially available from SILAB under the trade name of Detoxyl, a blend of ivy extract, phytic acid and sunflower seed extract (for example, product commercially available from SEDERMA under the trade name of OSMOPUR)), and the like.

Examples of the keratolytic agents include α-hydroxy acids (specifically, glycolic, lactic, citric, malic, mandelic or tartaric acid), β-hydroxy acids (specifically, salicylic acid), esters thereof (specifically, C12-13 alkyl lactate), and plant extracts containing these hydroxy acids (specifically, Hibiscus sabdriffa extracts), and the like.

Examples of the water-soluble polymer include dextrin, methylcellulose, ethylcellulose, sodium carboxymethylcellulose, hydroxyethyl cellulose, hydroxypropyl cellulose, hydroxypropylmethylcellulose stearoyl ester, PVA, PVM, PVP, locust bean gum, guar gum, tara gum, tamarind gum, glucomannan, xylan, mannan and agar.

Examples of the anti-inflammatory agents include bisabolol, allantoin, tranexamic acid, zinc oxide, sulfur oxide and its derivatives, chondroitin sulfate, and glycyrrhizic acid and its derivatives (for example, glycyrrhizinates).

The cosmetic composition of the present invention may contain at least one whitening agent to block the synthesis of structural proteins such as the melanocyte-specific protein Pmel17 involved in the mechanism of melanogenesis (stage I). Example of such a whitening agent may include the ferulic acid-containing cytovector (water, glycol, lecithin, ferulic acid, hydroxyethylcellulose) commercially available from BASF under the trade name of Cytovector [®].

Furthermore, if necessary, the cosmetic composition of the present invention may contain at least one peptide as described in International Publication WO2009/010356 pamphlet.

Furthermore, if necessary, the cosmetic composition of the present invention may include a whitening agent having an inhibition effect on melanin synthesis and/or an inhibition effect on nanophthalmia-related transcription factor (MITF) expression and/or an anti-tyrosinase activity and/or an inhibition effect on endothelin-1 synthesis. Examples of such a whitening agent include Glycyrrhiza glabra extract commercially available from Maruzen Pharmaceuticals Co. LTD. under the trade name of Licorice extract [®].

Furthermore, if necessary, the cosmetic composition of the present invention may include whitening agents having an antioxidant effect as well, such as vitamin C compounds, which include ascorbate salts, ascorbyl esters of fatty acids or of sorbic acid, and other ascorbic acid derivatives. Specific examples include ascorbyl phosphates (magnesium ascorbyl phosphate, sodium ascorbyl phosphate, and the like), and saccharide esters of ascorbic acid (ascorbyl-2-glucoside, 2-O-α-D-glucopyranosyl L-ascorbate, 6-O-β-D-galactopyranosyl L-ascorbate, and the like). Active agents of this type are commercially available from DKSH under the trade name of Ascorbyl glucoside [®].

Furthermore, if necessary, the cosmetic composition of the present invention may include other whitening agents. Examples of the other whitening agents may include pigmentation inhibiting agents such as plant extracts (e.g., Narcissus tazetta extracts), cetyl tranexamate (Nikko Chemicals Co., Ltd; trade name: NIKKOL TXC), arbutin, kojic acid, ellagic acid, cysteine, 4-thioresorcin, resorcinol or rucinol or their derivatives, glycyrrhizic acid, hydroquinone-β-glucoside, and the like.

Furthermore, if necessary, the cosmetic composition of the present invention may also include organic and/or inorganic sunscreens.

Examples of the organic sunscreens include dibenzoylmethane derivatives such as butyl methoxydibenzoylmethane (product commercially available from HOFFMANN LA ROCHE Ltd. under the trade name of Parsol 1789); cinnamic acid derivatives such as octyl methoxycinnamate (product commercially available from HOFFMANN LA ROCHE Ltd. under the trade name of Parsol MCX), salicylates, para-aminobenzoic acids; β,β'-diphenylacrylate derivatives; benzophenone derivatives; benzylidenecamphor derivatives such as terephthalylidene dicamphor sulphonic acid; phenylbenzimidazole derivatives; triazine derivatives; phenylbenzotriazole derivatives; anthranilic acid derivatives, and the like, all of which may be coated or encapsulated.

Examples of the inorganic sunscreens may include pigments or nanopigments formed from coated or uncoated metal oxides. Examples of the nanopigments include titanium oxide, iron oxide, zinc oxide, zirconium oxide or cerium oxide, which are all well known as UV photoprotective agents.

Examples of the antiseptic agent include p-hydroxybenzoate ester (e.g., methylparaben and propylparaben) and phenoxyethanol.

In addition, as additives to be used in the cosmetic composition of the present invention, those mentioned in International Cosmetic Ingredient Dictionary and Handbook, 16h Edition, 2016, published by the Personal Care Products Council, can be used.

The formulation of the cosmetic composition of the present invention is arbitrarily selectable, and a preferred formulation may be adopted according to the product form. For example, a formulation such as a solution type, an emulsion type, a powder dispersion type, a water-oil bilayer type, a water-oil-powder trilayer type, a gel type, and an oil type can be adopted.

The cosmetic composition of the present invention is used in the form of, for example, a make-up cosmetic (such as foundation, eye makeup such as an eye shadow, an eye shadow base, a blush, a mascara, a lip make-up product, a body make-up product, and a nail product) and a skin care cosmetic (such as an emulsion, a cream, and a sunscreen.

Among them, the cosmetic composition of the present invention is preferably used as a skin make-up cosmetic composition, particularly a foundation (such as a liquid foundation, a cream foundation, a solid foundation, a make-up base, a powder foundation), an eye shadow, a blush, a loose powder, a concealer, and the like.

In a yet preferred embodiment, the present invention is preferably a liquid foundation, a cream foundation, a solid foundation, a lip make-up, a concealer, an eye makeup.

In the preparation of these cosmetic compositions, the surface-treated pigment of the present invention may be additionally blended into a conventional formulation. The one skilled in the art knows how to prepare these cosmetic compositions.

Although the content of the surface-treated pigment varies depending on the purpose and usage, normally, it is preferably 0.1% by weight or more, more preferably 0.5% or more, more preferably 2% by weight or more, relative to the total weight of the cosmetic composition. Also, the content of the surface-treated pigment is preferably 30% by weight or less, more preferably 20% by weight or less, and even more preferably 15% by weight or less, relative to the total weight of the cosmetic composition.

When the surface-treated pigment is used in a cosmetic composition, it is prepared by a conventional method using other components. For example, liquid, emulsion, cream, solid, gel, paste, can be implemented in various form.

For example, when the cosmetic composition of the present invention is used as a liquid foundation (such O/W type), the content of the surface-treated pigment is preferably 0.5% by weight or more, more preferably 1% by weight or more, and even more preferably 2% by weight or more relative to the total weight of the cosmetic composition. Also, the content of the surface-treated pigment is preferably 30% by weight or less, more preferably 20% by weight or less, and even more preferably 15% by weight or less.

Also, for example, when the cosmetic composition of the present invention is used as a lip make-up product, the content of the surface-treated pigment is preferably 0.5% by weight or more, more preferably 1% by weight or more, and even more preferably 2% by weight or more relative to the total weight of the cosmetic composition. Also, the content of the surface-treated pigment is preferably 20% by weight or less, more preferably 15% by weight or less, and even more preferably 10% by weight or less.

Also, for example, when the cosmetic composition of the present invention is used as a concealer, the content of the surface-treated pigment is preferably 1% by weight or more, more preferably 3% by weight or more, and even more preferably 5% by weight or more relative to the total weight of the cosmetic composition. Also, the content of the surface-treated pigment is preferably 30% by weight or less, more preferably 20% by weight or less, and even more preferably 15% by weight or less.

## Use

Advantageously, the surface-treated pigments have improved stability of their color and shade over time, so that cosmetic compositions which include them remain stable as regard to the color and do not fade or discolor or migrate or change.

Hence, the present invention also relates, in a **fourth aspect** to the use of surface-treated pigment according to the present invention for improving the stability of the color and brightness of the make-up.

The surface-treated pigment is the surface-treated pigment as previously described. All the previously described embodiments are encompassed in this aspect.

### Colorimetric measurements (L*a*b system)

Color and brightness properties have been evaluated with colorimetric measurements (L*a*b* system).

Difference in color between dry and wet condition has been evaluated. The overall color change ($\Delta E$) was obtained by this formula:

$$\Delta E^*_{ab} = \sqrt{\left(L^*_2 - L^*_1\right)^2 + \left(a^*_2 - a^*_1\right)^2 + \left(b^*_2 - b^*_1\right)^2}$$

The higher the value of $\Delta E$ is, the greater the color changes.

Values of ∆E obtained with the surface-treated pigments according to the invention are lower than those obtained with surface-treated treatment according to the state of the art.

Advantageously, the treatment according to the present invention allows obtaining surface-treated pigment having improved stability properties, as regards to color and brightness overtime. Thus, the cosmetic compositions according to the invention remain stable as regard to the color and do not fade or discolor or migrate or change.

The surface-treated pigment hence improves the stability of the color and brightness of the make-up.

As used herein, make-ups having improved color stability and brightness properties refer to make-ups having a smaller ∆E ; it is understood that the range of ∆E is dependent of the color of the pigment itself.

The invention will be further illustrated by the following examples. However, these examples should not be interpreted in any way as limiting the scope of the present invention.

## EXAMPLES:

### Materials and Methods

### Dextrin isostearate

Dextrin isostearate used is the one manufactured by CHIBA FLOUR MILLING CO., Ltd, under the commercial name UNIFILMA HVY.

### Pigments

Iron oxides used are those manufactured by TITANIUM INDUSTRY CO., LTD, such as TAROXR-516HP or TAROXLL-100HP.

### Colorimetric measurements (L*a*b* system)

Colorimetric measurements have been made with a spectrophotometer (CM-2600d, manufactured by Konica Minolta).

The color change (∆E) was obtained by the following formula:

$$\Delta E_{ab}^* = \sqrt{(L_2^* - L_1^*)^2 + (a_2^* - a_1^*)^2 + (b_2^* - b_1^*)^2}$$

L* indicates lightness, a* is the red/green coordinate, and b* is the yellow/blue coordinate.

Deltas for L* (∆L*), a* (∆a*) and b* (∆b*) may be positive (+) or negative (-). The total difference, Delta E (∆E*), however, is always positive.

∆L* ($L_2$* sample minus $L_1$* standard) = difference in lightness and darkness (+ = lighter, - = darker)

∆a* ($a_2$* sample minus $a_1$* standard) = difference in red and green (+ = redder, - = greener)

∆b* ($b_2$* sample minus $b_1$* standard) = difference in yellow and blue (+ = yellower, - = bluer)

### Example 1: Production of surface-treated pigments according to the invention

In the present example, pigments have been treated with acrylates/dimethicone copolymer and dextrin isostearate.

### A. Treatment of a red iron oxide with acrylates/dimethicone copolymer and dextrin isostearate

The red iron oxide (TAROXR-516HP: manufactured by Titanium Industry Co., Ltd.) was treated with 5% of acrylates/dimethicone copolymer and 10% of dextrin isostearate.

The treatment method was as follows:

The 8.3 pts.wt.(parts by weight) of acrylates/dimethicone copolymer (active ingredient 60% product) and the 10 pts.wt. of dextrin isostearate were dissolved in the 16.5 pts.wt. of hexane (solvent), and they were added to the 85 pts.wt. of the pigment during stirring and mixing with a Henschel mixer. After stirring and mixing, hexane was removed and dried, and the pigment was pulverized with a pulverizer, and then finally they were heat-treated to obtain the surface-treated pigment.

### B. Treatment of a yellow iron oxide with acrylates/dimethicone copolymer and dextrin isostearate

The yellow iron oxide (TAROXLL-100HP: manufactured by Titanium Industry Co., Ltd.) was treated with 7% of acrylates/dimethicone copolymer and 10% of dextrin isostearate.
The treatment method was as same as above Example 1.A except 7% of acrylates/dimethicone copolymer and 10% of dextrin isostearate.

### C. Treatment of white pigment, titanium dioxide with acrylates/dimethicone copolymer and dextrin isostearate

The white pigment, titanium dioxide (CR-50: manufactured by ISHIHARA SANGYO KAISHA, LTD.) was treated with 2% of acrylates/dimethicone copolymer and 2% of dextrin isostearate.
The treatment method was as same as above Example 1.A except 2% of acrylates/dimethicone copolymer and 2% of dextrin isostearate.

### Example 2: Production of dextrin isostearate surface-treated pigments according to the state of the art

According to one of the methods of the state of the art, pigments were treated with dextrin isostearate.

### A. Coating of red iron oxide with dextrin isostearate

The red iron oxide (TAROXR-516HP: manufactured by Titanium Industry Co., Ltd.) was treated with 10% of dextrin isostearate.
The treatment method was as same as above Example 1.A except only 10% of dextrin isostearate.

### B. Coating of yellow iron oxide with dextrin isostearate

The yellow iron oxide (TAROXLL-100HP: manufactured by Titanium Industry Co., Ltd.) was treated with 10% of dextrin isostearate.
The treatment method was as same as above Example 1.A except only 10% of dextrin isostearate.

### C. Coating of white pigment, titanium dioxide with dextrin isostearate

The white pigment, titanium dioxide (CR-50: manufactured by ISHIHARA SANGYO KAISHA, LTD.) was treated with 2% of dextrin isostearate.
The treatment method was as same as above ASC-5 HVY-10 RED R-516P except only 2% of dextrin isostearate.

### Example 3: Surface-treated pigments according to the state of the art

In the following examples, acrylates/dimethicone copolymers used are those manufactured by DAITO KASEI KOGYO CO., LTD under the product name "ASC treatment" and the following commercial references :

### A. Red pigment iron oxide coated with acrylates/dimethicone copolymer

The red pigment, iron oxide treated by acrylates/dimethicone copolymer has the commercial reference ASC-7 YELLOW LL-100P.

### B. Coating of yellow iron oxide coated with acrylates/dimethicone copolymer

The yellow pigment, iron oxide treated by acrylates/dimethicone copolymer has the commercial reference ASC-5 RED R-516L.

### C. Coating of white pigment titanium dioxide coated with acrylates/dimethicone copolymer

The white pigment, titanium treated by acrylates/dimethicone copolymer has the commercial reference ASC-2 TiO2 CR-50.

### Example 4: Difference in color between dry and wet condition of treated pigments

One principal cause for the evolution of the color composition is the difference of color between pigments when dispersed in a wet formulation compared to its dry form.

Hence, measurements were made in order to evaluate the difference in color between dry and wet conditions of treated pigments.

Test method to measure difference in color using L*a*b* system was as follows:

#### a. Dry condition measurement:

Each 2g of pigment listed in the table below was placed on the filter paper (No.5C, 150mm diameter) and the filter paper was folded in half.

Then, the pigment sandwiched between the filter papers was compressed.

And then the compressed pigment color was measured by a spectrophotometer (CM-2600d, manufactured by Konica Minolta).

#### b. Wet condition measurement:

Each 2g of pigment listed in the table below was placed on the filter paper (No.5C, 150mm diameter) and the pigment is wetted by squalane.

The surface of the wetted pigment was smoothed with a spatula.

Then the wetted pigment color was measured by a spectrophotometer (CM-2600d, manufactured by Konica Minolta).

#### c. Difference in color between dry and wet condition:

The difference in color between the wet state and the dry state of each pigment was compared table 1.

*Table 1: Difference in color between dry and wet condition*

|  | Comparative | | | Invention |
|---|---|---|---|---|
|  | Red iron oxide, TAROXR-516HP | Example 3.A | Example 2.A | **Example 1.A** |
| dL* | 3.8 | 5.91 | 3.58 | **1.55** |
| da* | 12.03 | 11.61 | 10.42 | **8.56** |
| db* | 14.41 | 14.1 | 8.5 | **6.54** |
| ΔE* | 19.15 | 19.20 | 13.92 | **10.88** |

Treated red pigments according to the present invention (Example 1.A) have lower value of ΔE compared to surface treated pigments according to the state of the art, i.e., red pigment coated with dextrin isostearate (example 2A) and red pigment coated with acrylates/dimethicone copolymer (example 3A).

| | Comparative | | | Invention |
|---|---|---|---|---|
| | Yellow iron oxide, TAROXLL-100HP | Example 3.B | Example 2.B | **Example 1.B** |
| dL* | 13.4 | 12.55 | 13.9 | **1.38** |
| da* | 1.79 | 2.74 | 1.29 | **3.76** |
| db* | 11.09 | 22.9 | 12.94 | **7.03** |
| ΔE* | 17.49 | 26.26 | 19.03 | **8.09** |

Treated yellow pigments according to the present invention (Example 1.B) have lower value of ΔE compared to surface treated pigments according to the state of the art, i.e., yellow pigment coated with dextrin isostearate (example 2B) and yellow pigment coated with acrylates/dimethicone copolymer (example 3B).

In red and yellow pigments, the ASC HVY treated pigments according to the present invention (Examples 1.A and 1.B) have lower value of ΔE than other pigments. It shows the pigments are already wetted, by the dextrin isostearate, before adding oil (squalane). They hence have improved color stability and brightness properties refer to make-ups havin

### Example 5: Difference in color change when emulsified formula

Test method to measure difference in color using L*a*b system was as follows:

a. *Water-in-oil type emulsions formulas*:

This invention of water-in-oil emulsions were prepared in accordance with formulations shown in Table 2, as follows:
Components 1 to 4 were stirred until homogeneous to obtain an oil phase mixture.

Separately, components 5 to 8 were stirred until homogeneous to obtain an aqueous phase mixture.

And then, to the oil phase mixtures, the aqueous phase mixture was added with stirring to obtain a water-in-oil emulsion.

Finally, to the water-in-oil emulsion, each pigment was added and mixed well.

*Table 2: water-in-oil type emulsion formula with ASC HVY treated pigment, Examples* 1

| | | | Invention | | |
|---|---|---|---|---|---|
| 1 | | Surfactant: Lauryl PEG-9 Polydimethylsiloxyethyl Dimethicone (KF-6038: Shin-Etsu Chemical Co., Ltd.) | 1.0 | 1.0 | 1.0 |
| 2 | | Preservative: Caprylic Glycol | 0.2 | 0.2 | 0.2 |
| 3 | | Oil 1: Squalane | 10.0 | 10.0 | 10.0 |
| 4 | | Oil 2: Isododecane | 9.39 | 9.16 | 10.47 |
| 5 | | Glycerin | 6.0 | 6.0 | 6.0 |
| 6 | | Ethanol | 6.0 | 6.0 | 6.0 |
| 7 | | Water | 57.7 | 57.7 | 57.7 |
| 8 | | Magnesium sulfate | 0.3 | 0.3 | 0.3 |
| 9 | | Example 1.A* | 9.41 | - | - |
| | | Example 1.B* | - | 9.64 | - |
| | | Example 1.C* | - | - | 8.33 |

(continued)

| | Invention | | |
|---|---|---|---|
| Total (wt%) | 100 | 100 | 100 |

\* Each treated pigment was added so that the iron oxide or titanium dioxide content was 8 wt%.

b. *Wet condition measurement:*

The tested emulsions were drawn on paper (byko-charts, Uncoated Chart #2831) with 200 $\mu$m thickness coater. The chart has white part and black part, and the color was measured by a spectrophotometer on the black part (CM-2600d, manufactured by Konica Minolta).

c. *Dry condition measurement:*

The $\Delta E$ were measured after drying completely by spectrophotometer.

d. Difference in color between dry and wet condition:

The differences in color between the wet condition and the dry condition of each pigment were compared Table 3.

*Table 3: Difference in color between dry and wet condition, $\Delta E$*

| Comparative | | Invention |
|---|---|---|
| Example 3.A | Example 2.A | **Example 1.A** |
| 4.65 | 3.50 | **2.39** |
| Example 3.B | Example 2.B | **Example 1.B** |
| 8.01 | 5.90 | **5.36** |
| Example 3.C | Example 2.C | **Example 1.C** |
| 7.48 | 9.24 | **6.51** |

ASC HVY treated pigments of red iron oxide, yellow pigments and white pigments (Examples 1.A; 1.B; 1.C) had lower value of $\Delta E$, compared to surface treated pigments according to the state of the art, i.e., pigment coated with dextrin isostearate (examples 2A, 2B and 2C) and pigment coated with acrylates/dimethicone copolymer (example 3A, 3B and 3C).

Hence, and according to the present invention, the treated pigments have improved stability properties, as regards to color and brightness overtime. Indeed, the pigments are wetted by the dextrin isostearate, the oil is hence adsorbed around the pigments. As the pigments' surface is also treated with acrylates/dimethicone copolymer, said acrylates/dimethicone copolymer prevents elution of the dextrin isostearate. The oil is thus maintained around the pigment to keep the pigment in a wet state for a long period of time.

Hence, and advantageously, the cosmetic composition containing such pigments remain stable as regard to the color and do not fade or discolor or migrate of change.

The wetting of the pigment in a specific oil through a first coating and an entrapment of said wetted pigments through another specific second coating hence improved color stability and brightness of the make -up.

**Claims**

1. A surface-treated pigment **characterized in that** its surface-treatment comprises at least a dextrin fatty acid ester and at least a silicone acrylate copolymer,

   wherein the pigment is a mineral pigment, and
   wherein the surface-treatment comprises 1 to 15 % by weight, relative to the total weight of the composition, of the dextrin fatty acid ester and 1 to 15% by weight relative to the total weight of the composition, of the silicone acrylate copolymer.

15

2. A surface-treated pigment according to claim 1, wherein the dextrin fatty acid ester is a dextrin isostearate.

3. A surface-treated pigment according to claim 1 or 2, wherein the silicone acrylate is an acrylates/dimethicone copolymer.

4. A surface-treated pigment according to any one of claims 1 to 3, wherein the mineral pigment is selected from the group consisting of zirconium oxide, cerium oxide, zinc oxide, iron oxide, chromium oxide, manganese violet, ultramarine blue, chromium hydrate, ferric blue, titanium dioxide and metal powders.

5. A surface treated pigment according to claim 4, wherein the mineral pigment is an iron oxide.

6. A surface-treated pigment according to any one of claims 1 to 5, wherein the surface-treatment comprises 2 to 10 % by weight, relative to the total weight of the composition, of the dextrin fatty acid ester and 2 to 7% by weight relative to the total weight of the composition, of the silicone acrylate copolymer.

7. A process of preparing a surface-treated pigment according to any one of claims 1 to 6 comprising:

   - dissolving a dextrin fatty ester and a silicone acrylate copolymer in a solvent to form a slurry;
   - adding the slurry to pigments under stirring;
   - removing the solvent;
   - Pulverizing and heating the pigments in order to obtain surface-treated pigments.

8. A cosmetic composition comprising a surface-treated pigment according to any one of claims 1 to 6 and a cosmetically acceptable medium.

9. The cosmetic composition according to claim 8, wherein the cosmetic composition is selected among a liquid foundation, a cream foundation, a solid foundation, a lip make-up, an eye makeup and a concealer.

10. The use of surface-treated pigment according to any one of claims 1 to 6 for improving the stability of the color and brightness of the make-up.


**Patentansprüche**

1. Oberflächenbehandeltes Pigment, **dadurch gekennzeichnet, dass** seine Oberflächenbehandlung wenigstens einen Dextrinfettsäureester und wenigstens ein Siliconacrylat-Copolymer umfasst,

   wobei das Pigment ein Mineralpigment ist, und
   wobei die Oberflächenbehandlung 1 bis 15 Gew.-%, bezogen auf das Gesamtgewicht der Zusammensetzung, an dem Dextrinfettsäureester und 1 bis 15 Gew.-%, bezogen auf das Gesamtgewicht der Zusammensetzung, an dem Siliconacrylat-Copolymer umfasst.

2. Oberflächenbehandeltes Pigment nach Anspruch 1, wobei der Dextrinfettsäureester ein Dextrinisostearat ist.

3. Oberflächenbehandeltes Pigment nach Anspruch 1 oder 2, wobei das Siliconacrylat ein Acrylat-Dimethicon-Copolymer ist.

4. Oberflächenbehandeltes Pigment nach einem der Ansprüche 1 bis 3, wobei das Mineralpigment ausgewählt ist aus der Gruppe bestehend aus Zirkoniumoxid, Ceroxid, Zinkoxid, Eisenoxid, Chromoxid, Manganviolett, Ultramarinblau, Chromhydrat, Eisenblau, Titandioxid und Metallpulvern.

5. Oberflächenbehandeltes Pigment nach Anspruch 4, wobei das Mineralpigment ein Eisenoxid ist.

6. Oberflächenbehandeltes Pigment nach einem der Ansprüche 1 bis 5, wobei die Oberflächenbehandlung 2 bis 10 Gew.-%, bezogen auf das Gesamtgewicht der Zusammensetzung, an dem Dextrinfettsäureester und 2 bis 7 Gew.-%, bezogen auf das Gesamtgewicht der Zusammensetzung, an dem Siliconacrylat-Copolymer umfasst.

7. Verfahren zur Herstellung eines oberflächenbehandelten Pigments nach einem der Ansprüche 1 bis 6, umfassend:

- Lösen eines Dextrinfettesters und eines Siliconacrylat-Copolymers in einem Lösungsmittel, um eine Aufschlämmung zu bilden;
- Zugeben der Aufschlämmung zu Pigmenten unter Rühren;
- Entfernen des Lösungsmittels;
- Pulverisieren und Erhitzen der Pigmente, um oberflächenbehandelte Pigmente zu erhalten.

8. Kosmetische Zusammensetzung umfassend ein oberflächenbehandeltes Pigment nach einem der Ansprüche 1 bis 6 und ein kosmetisch annehmbares Medium.

9. Kosmetische Zusammensetzung nach Anspruch 8, wobei die kosmetische Zusammensetzung ausgewählt ist aus einer flüssigen Grundlage, einer Cremegrundlage, einer festen Grundlage, einem Lippen-Make-up, einem Augen-Make-up und einem Concealer.

10. Verwendung von oberflächenbehandeltem Pigment nach einem der Ansprüche 1 bis 6 zur Verbesserung der Stabilität der Farbe und Helligkeit des Make-up.

**Revendications**

1. Pigment traité en surface **caractérisé en ce que** son traitement en surface comprend au moins un ester d'acide gras de dextrine et au moins un copolymère acrylate de silicone,

   dans lequel le pigment est un pigment minéral, et
   dans lequel le traitement en surface comprend 1 à 15 % en poids, par rapport au poids total de la composition, de l'ester d'acide gras de dextrine et 1 à 15 % en poids par rapport au poids total de la composition, du copolymère d'acrylate de silicone.

2. Pigment traité en surface selon la revendication 1, dans lequel l'ester d'acide gras de dextrine est un isostéarate de dextrine.

3. Pigment traité en surface selon la revendication 1 ou 2, dans lequel l'acrylate de silicone est un copolymère acrylates/diméthicone.

4. Pigment traité en surface selon l'une quelconque des revendications 1 à 3, dans lequel le pigment minéral est choisi dans le groupe constitué par l'oxyde de zirconium, l'oxyde de cérium, l'oxyde de zinc, l'oxyde de fer, l'oxyde de chrome, le violet de manganèse, le bleu outremer, l'hydrate de chrome, le bleu ferrique, le dioxyde de titane et les poudres métalliques.

5. Pigment traité en surface selon la revendication 4, dans lequel le pigment minéral est un oxyde de fer.

6. Pigment traité en surface selon l'une quelconque des revendications 1 à 5, dans lequel le traitement en surface comprend 2 à 10 % en poids, par rapport au poids total de la composition, de l'ester d'acide gras de dextrine et 2 à 7 % en poids, par rapport au poids total de la composition, du copolymère d'acrylate de silicone.

7. Procédé de préparation d'un pigment traité en surface selon l'une quelconque des revendications 1 à 6 comprenant :

   - dissolution d'un ester gras de dextrine et d'un copolymère d'acrylate de silicone dans un solvant pour former une suspension ;
   - ajout de la suspension aux pigments sous agitation ;
   - élimination du solvant ;
   - pulvérisation et chauffage des pigments afin d'obtenir des pigments traités en surface.

8. Composition cosmétique comprenant un pigment traité en surface selon l'une quelconque des revendications 1 à 6 et un milieu cosmétiquement acceptable.

9. Composition cosmétique selon la revendication 8, dans laquelle la composition cosmétique est choisie parmi un fond de teint liquide, un fond de teint crème, un fond de teint solide, un maquillage pour les lèvres, un maquillage pour les yeux et un anticerne.

10. Utilisation d'un pigment traité en surface selon l'une quelconque des revendications 1 à 6 pour améliorer la stabilité de la couleur et de la brillance du maquillage.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5695331 B
- JP 2011213662 A
- KR 20180136315
- WO 2018029209 A
- EP 2537865 A
- FR 2679771
- EP 1184426 A
- WO 2009010356 A

**Non-patent literature cited in the description**

- Inorganic Pigment. Ullmann Encyclopedia